# EUROPEAN PATENT SPECIFICATION

(11) **EP 4 362 995 B1**
(45) Date of publication and mention of the grant of the patent: **11.11.2026**
(21) Application number: 22738799.0
(22) Date of filing: 06.06.2022
(51) Int. Cl.: A61L 29/02, A61L 29/14

(54) **PLASMA SURFACE TREATMENT FOR INTRAVASCULAR SYSTEMS**
PLASMAOBERFLÄCHENBEHANDLUNG FÜR INTRAVASKULÄRE SYSTEME
TRAITEMENT DE SURFACE PAR PLASMA POUR SYSTÈMES INTRAVASCULAIRES

(30) Priority: 29.06.2021 US 202163216270 P
(43) Date of publication of application: 08.05.2024
(73) Proprietor: Covidien LP, Mansfield, MA 02048 (US)
(72) Inventor: SONG, Kevin, Irvine, California 92617 (US)
(74) Representative: Gray, James
(86) International application number: PCT/US2022/032359
(87) International publication number: WO 2023/278103

(56) References cited:
- WO-A1-2013/031459
- US-B2- 10 016 533
- GRONING P ET AL: "Surface modifications of nitrogen-plasma-treated stainless steels", APPLIED SURFACE SCIENCE,, vol. 64, no. 3, 1 March 1993 (1993-03-01), pages 265 - 273, XP024428687, [retrieved on 19930301], DOI: 10.1016/0169-4332(93)90033-8
- E. ABUSRAFA AYA ET AL: "Modification of Polyethylene by RF Plasma in Different/Mixture Gases", COATINGS, vol. 9, no. 2, 22 February 2019 (2019-02-22), pages 145, XP055954901, DOI: 10.3390/coatings9020145

## Description

### TECHNICAL FIELD

The disclosure relates to intravascular medical devices and systems.

### BACKGROUND

Intravascular medical assemblies, such as medical devices and their associated delivery or retrieval systems, may be used, e.g., to occlude a vasculature of a patient or to clear occlusions from the vasculature of the patient, as appropriate. For instance, an implantable embolization device may be configured to fill a vascular site in the patient, thereby reducing blood flow, promoting clotting, and eventually occluding the vessel. Some example clinical applications thereof include controlling bleeding from hemorrhages, reducing blood flow to tumors, and treating aneurysms, vascular malformations, arteriovenous fistulas, pelvic congestion syndrome, and varicoceles. In other examples, an aspiration catheter may be advanced through a patient's vasculature to remove a thrombus or other occlusive material obstructing the flow of the patient's blood.

### SUMMARY

In general, this disclosure describes intravascular medical assemblies, including intravascular medical devices, intravascular medical-device delivery systems, and/or medical-device retrieval systems. In examples described herein, such assemblies include at least one exposed surface comprising a metallic surface, treated with a customized plasma-treatment process configured to facilitate or improve the delivery, placement, retention, and/or retrieval of an intravascular medical device, by reducing friction associated with the surface, and wherein the exposed surface includes a hydrophilic substance formed from the reactive gas, wherein the plasma-treatment process comprises electrolytic-polishing of the surface in the presence of the reactive gas to reduce variability in the surface, wherein the reactive gas comprises a mixture of nitrogen and oxygen, wherein the hydrophilic substance comprises one or more groups comprising nitrogen and oxygen, and wherein the mixture of nitrogen and oxygen comprises a nitrogen-oxygen ratio of greater than or equal to about 4: 1.

In one example of the medical assemblies described herein, an exposed surface of the medical assembly may be treated with a plasma-treatment process configured to impart hydrophilic properties onto the surface, e.g., in order to reduce friction associated with delivery or retrieval of a medical device through vasculature of a patient toward a target treatment site. In additional or alternative examples, an exposed surface may be treated with a different plasma-treatment process configured to impart hydrophobic properties onto the surface, e.g., in order to help retain the medical device at the target treatment site.

This disclosure also describes example plasma-based treatment techniques for producing, and example methods of using, the medical assemblies described herein. The details of one or more examples are set forth in the accompanying drawings and the description below. Other features, objects, and advantages will be apparent from the description an d drawings, and from the claims.

US10016533 provides an implantable medical device having at least one contacting surface for contacting a bodily fluid or tissue, wherein the contacting surface is coated by a two step process of plasma treatment comprising a first step of a plasma deposition process using silicon-containing monomers to provide a uniform and conformal nano-scale plasma coating and a second step of a plasma modification process using a mixture of nitrogen and oxygen molecules.

WO2013/031459 provides a medical device that exhibits lubricity and/or antithrombotic properties when wet. The medical device comprises a base layer and a hydrophilic layer supported on at least a part of the base layer. The hydrophilic layer is obtained by reacting a compound having multiple thiol groups within its molecule with a compound having a reactive functional group that binds to the thiol groups and a hydrophilic group within its molecule, thereby forming a cross-linked structure. The reactive functional group is characterized as being a thiol group, an alkenyl group, an acrylic group, or a methacrylic group and these reactive functional groups are highly reactive by treatments such as heating, ultraviolet irradiation, and plasma irradiation

### BRIEF DESCRIPTION OF DRAWINGS

FIG. 1 is a conceptual plan view illustrating an example intravascular-medical-device system, including an example embolization device and an example delivery device.
FIG. 2 is a conceptual plan view illustrating an example delivery assembly including the embolization device and the delivery device of FIG. 1, as well as a delivery catheter.
FIG. 3 is a conceptual transparent view illustrating the example delivery assembly of FIG. 2 positioned within a human body.
FIG. 4 is conceptual a cross-sectional view of a portion of vasculature of a patient, and the example delivery assembly of FIG. 2 positioned near a target site within the vasculature.
FIG. 5 is conceptual a cross-sectional view of the portion of the vasculature of the patient of FIG. 4, and illustrates the embolization device of FIG. 1 deployed at the target site.
FIG. 6 is a plan view, including cross-sectional views, illustrating an example system including an example embolization device and an example delivery device.
FIG. 7 is a flow diagram illustrating an example method of plasma-treating a medical assembly.
FIG. 8 is a flow diagram illustrating an example method of using a plasma-treated medical assembly.

### DETAILED DESCRIPTION

Medical assemblies may include intravascular medical devices (IMDs), as well as delivery systems configured to introduce the IMDs into, and retrieval systems configured to retrieve the IMDs from within, vasculature of a patient. As one illustrative example, an intravascular medical assembly may include an IMD configured to occlude a portion of the patient's vasculature. For instance, an implantable embolization device (IED) may be configured to fill a vascular site in the patient, thereby reducing local blood flow, promoting clotting, and eventually occluding the vessel. Some example clinical applications of such devices include controlling bleeding from hemorrhages, reducing blood flow to tumors, and treating aneurysms, vascular malformations, arteriovenous fistulas, pelvic congestion syndrome, and varicoceles.

In other examples, an intravascular medical assembly may include an IMD configured to reduce or remove occlusions from the patient's vasculature. For instance, an IMD may include an aspiration catheter and/or a mechanical thrombus-retrieval device configured to be advanced through the vasculature to remove a thrombus or other occlusive material obstructing the flow of the patient's blood.

In general, this disclosure describes various example medical assemblies, including IMDs, IMD-delivery systems, and/or IMD-retrieval systems, that include one or more surfaces that have been treated with at least one plasma-based surface-treatment process configured to facilitate or otherwise improve the delivery, placement, retention, or retrieval of an IMD within a patient's vasculature. The plasma-treatment processes may be selectively applied to particular portions of the medical assemblies to improve properties that are specific to the particular portions, such as a plasma-treatment to improve lubricity at portions of the medical assembly subject to friction or a plasma-treatment to increase thrombogenicity at portions of the medical assembly used for clotting. Such plasma-treatment processes may result in surface treatments, such as surface modifications (e.g., roughness or smoothness), surface functionalizations (e.g., hydroxyl reactive groups), or surface coatings (e.g., nitriding or oxiding layer), that may have reduced likelihood of delamination compared to polymerized coatings, thereby reducing a risk of the coating being released into the vasculature of the patient.

The plasma-treatment processes may impart a degree of polarity (e.g., polar/hydrophilic, nonpolar/hydrophobic, or intermediates thereof) and/or a degree of roughness to configure the one or more surfaces of the medical assemblies with one or more properties that may affect delivery, retrieval, retention, or operation of the medical assembly in a patient, such as lubricity, antithrombogenicity, thrombogenicity, or anti-fouling. For example, a medical assembly may include an elongated structure defining an exposed surface that is plasma-treated so as to impart generally hydrophilic properties onto the surface, e.g., in order to reduce friction associated with delivery or retrieval of an IMD through the patient's vasculature. Additionally or alternatively, a medical assembly may include a surface that is plasma-treated so as to impart generally hydrophobic properties onto the surface. For instance, a plasma-treated hydrophobic surface of an IMD may exhibit improved thrombogenic properties, e.g., by promoting fibrin growth, thereby inducing the patient's blood to clot around the IMD to help retain the IMD at a desired target treatment site within the patient's vasculature.

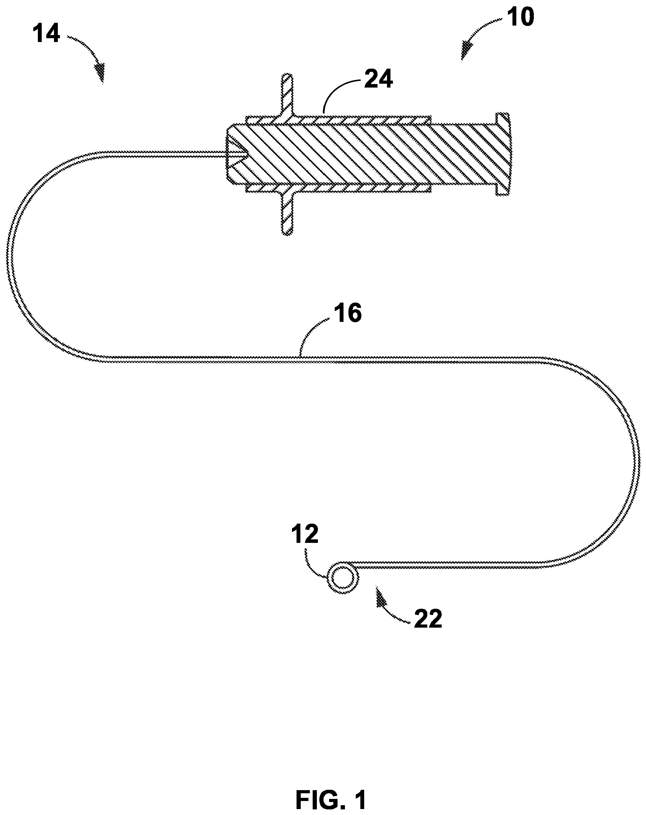

The plasma-based surface-treatment techniques of this disclosure are primarily described with respect to a medical assembly that includes an embolization device, such as described in FIGS. 1-6. For example, an embolization device may contact various friction surfaces during delivery and contact bodily fluids and tissues during operation, such that plasma-treatment processes may be particularly useful for customizing various surfaces of the medical assembly for improving delivery and/or operation. However, this description is intended to be illustrative in nature, and is not intended to be limiting; the plasma-based surface-treatment techniques described herein may be similarly applied to virtually any suitable intravascular medical assembly for the generally applicable purposes (e.g., benefits, advantages, and practical applications) detailed further below.

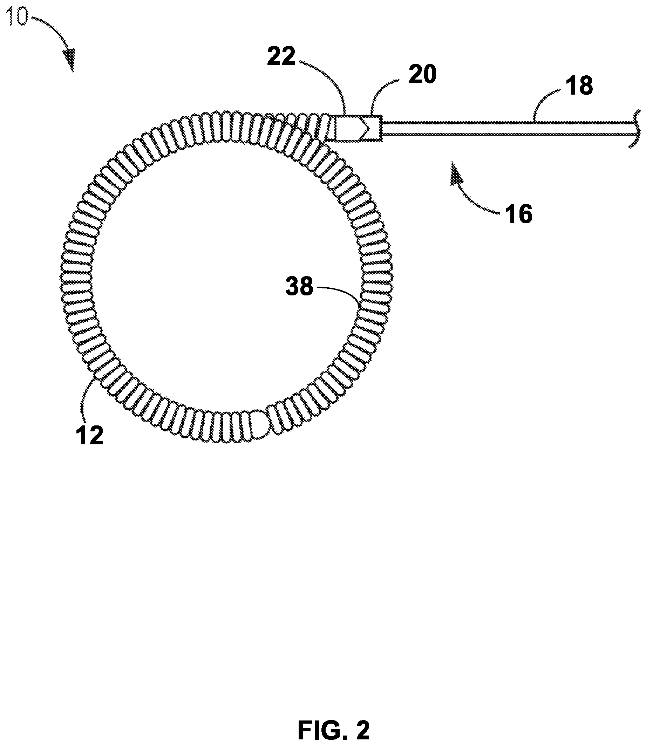

FIG. 1 is a conceptual diagram illustrating an example medical assembly 10, and FIG. 2 illustrates a distal portion of the medical assembly 10 of FIG. 1. The medical assembly 10 includes an intravascular medical device (IMD) 12 (also referred to herein as an "embolization device 12"), and an IMD-delivery system 14 configured to deliver the IMD 12 through vasculature of a patient toward a target treatment site within the vasculature.

The IMD-delivery system 14 includes an elongated structure or elongated body 16, defining an elongated body proximal portion 18 and an elongated body distal portion 20. In the example depicted in FIGS. 1-6, the IMD-delivery system 14 includes a coil-delivery system, as described in further detail below. However, the techniques of this disclosure may apply to any suitable intravascular medical assembly 10.

For instance, the IMD-delivery system 14 may include a stent-delivery system, a flow-diverter-delivery system, a cardiac-pacing-device-delivery system, or a heart-valve-delivery system. In other examples, the IMD 12 may include an intravascularinterventional element, and the elongated body 16 of the delivery system 14 may include an intravascular-insertable shaft removably coupled to the interventional element. For instance, the IMD 12 may include a thrombectomy device or a clot-grabbing device positioned at the elongated body distal portion 20. In some examples, the IMD 12 may include a balloon-expandable interventional device. Additionally or alternatively, the IMD 12 may include an energy-emitting device or an energy-delivery device. For instance, the IMD 12 may include an electrode, a heating coil, a fiber-optic device, an electrical source, or an ultrasonic source.

Regardless of the particular type of the intravascular medical assembly 10, the elongated body 16 of the IMD-delivery system 14 defines a shaft length configured to enable neurovascular access, coronary access, cardiac access, or peripheral access for the elongated structure 16, as appropriate. For instance, the shaft length of elongated body 16 may be greater than one meter, such as about 160 centimeters (cm). The elongated body 16 may include one or more of a core wire, a hypotube, or a filament. In examples in which the elongated body 16 includes a hypotube, the elongated body 16 may be or may include a laser-cut hypotube, a spiral-cut hypotube, or a slotted-cut hypotube, as appropriate.

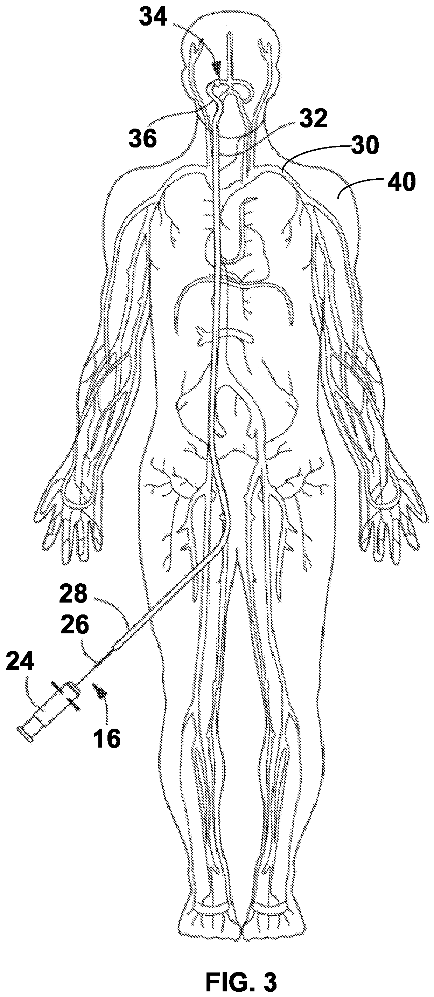

The intravascular medical assembly 10 further includes an interface member 22 engaging the elongated body distal portion 20 with the IMD 12. As shown in FIG. 1, the medical assembly 10 may further include an actuator 24. During use, a clinician may manipulate a longitudinal position of the embolization device 12 relative to a delivery catheter 26 (FIG. 3) using the actuator 24.

The medical assembly 10 may be introduced and navigated through vasculature of a patient to a target site within the vasculature using any suitable technique. In some examples, a clinician may use a guide tube 28 (also referred to herein as "outer catheter 28" or "sheath 28") to position the catheter 26 within vasculature 30 of a patient 40, as illustrated in FIG. 3. For example, the clinician may introduce the guide tube 28 into the patient's vasculature through an access point such as the groin, e.g., with the aid of a guidewire, and a direct distal end 32 of the guide tube 28 through the vascular system until it reaches the proximity of the target site 34. The clinician may then introduce the catheter 26 into the guide tube 28 and advance the catheter 26 through the guide tube 28 until the distal end 36 of the catheter 26 exits the distal end 32 of the guide tube 28 and is positioned near the target site 34.

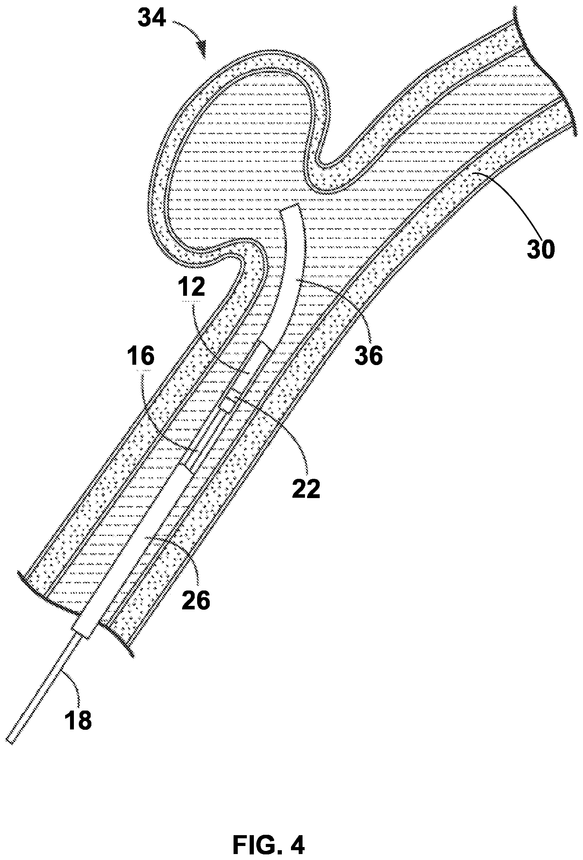

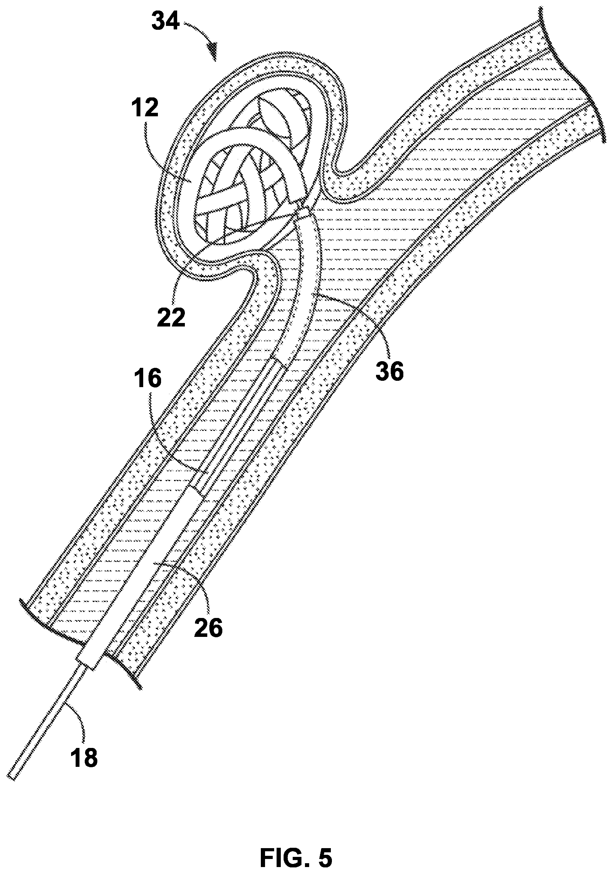

The clinician may deliver the embolization device 12 to the target site 34 by inserting the embolization device 12, the interface member 22, and the elongated body 16 into the catheter 26, and advancing the elongated body 16, the interface member 22, and the embolization device 12 toward the distal end 36 of the catheter 26 with a pushing force administered to the elongated body proximal portion 18. During navigation, various exposed surfaces of components of the medical assembly 10, such as exposed surfaces of the embolization device 12, the catheter 26, and/or the guide tube 28, may encounter resistance due to friction, such as with the vasculature 30 of the patient 40 or with one or more surfaces of the components of the medical assembly 10. This example mode of implant delivery is illustrated at FIGS. 4 and 5. The embolization device 12 may exhibit differing dimensions depending upon its surrounding environment. For example, a differing dimension of the embolization device 12 may include a primary dimension when the embolization device 12 is in a "delivery" configuration while being delivered through an inner lumen of the catheter 26, and a secondary dimension once the embolization device 12 is in a "deployed" configuration at the target site 34 (shown in FIG. 5). In some examples, the embolization device 12 is configured to self-expand from the delivery configuration to the deployed configuration in response to being released from the catheter 26. As illustrated in FIG. 5, while in the deployed configuration, the embolization device 12 is configured to fill the target site 34 and contact, through one or more exposed surfaces of the embolization device 12, biological fluids (e.g., blood) at or surfaces (e.g., tissues of the target site 34) of the target site 34, thereby reducing blood flow, promoting clotting, and eventually, occluding the vessel. The embolization device 12 can include, for example, a coil 38 (FIG. 2), such as, but not limited to, a framing coil, an anchoring coil, and/or a packing coil. Embolization devices such as the IMD 12 have been utilized in this manner for the treatment of hemorrhaging, aneurysms, and a multitude of diverse vascular pathologies, including malignancies, vascular. malformations, arteriovenous fistulas, pelvic congestion syndrome, and varicoceles.

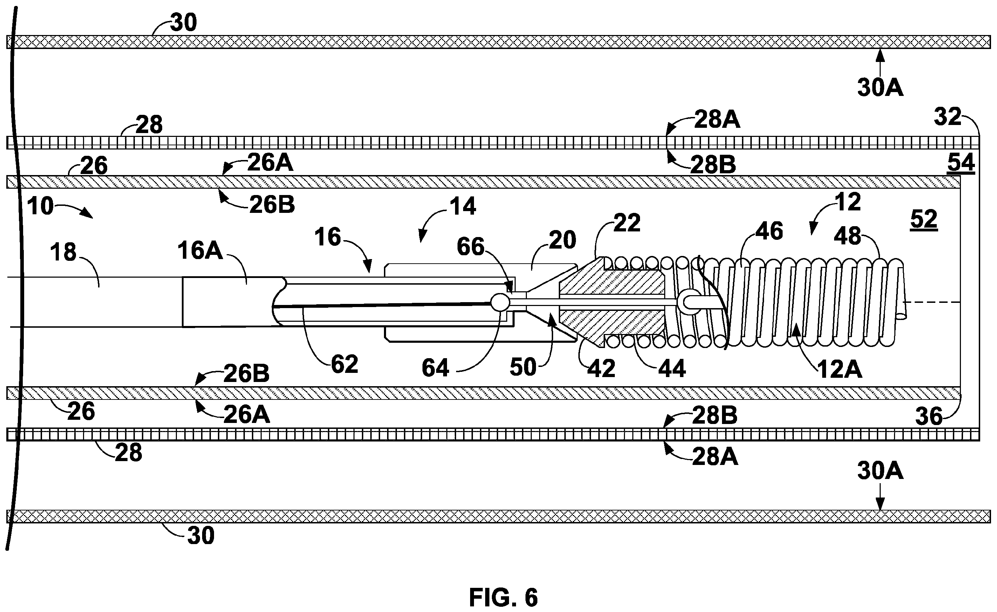

In accordance with techniques of this disclosure, the medical assembly 10 includes one or more exposed surfaces, which is an exposed metal surface that has been treated with a plasma-based treatment process prior to introduction of the medical assembly 10 into the vasculature of the patient. The plasma-based treatment processes described herein may similarly be applied to exposed surface(s) of any suitable medical assembly for the applications and purposes detailed below. For instance, FIG. 6 is a plan view, including cross-sectional views, illustrating an example of the medical system 10 of FIG. 1, e.g., deployed within the vasculature 30 of the patient 40 (FIG. 3).

More specifically, FIG. 6 is a side view illustrating an example of the medical assembly 10 including the elongated body 16, the interface member 22, and the IMD (e.g., embolization device) 12, and illustrates in further detail the relationships among the elongated body 16, the interface member 22, and the embolization device 12. The interface member 22 includes an interface member proximal portion 42 and an interface member distal portion 44, and the embolization device 12 includes a device proximal portion 46 and a device distal portion 48. The device proximal portion 46 of the embolization device 12 is mechanically connected to the interface member 22, e.g., to the interface member distal portion 44, and the embolization device 12 is further attached to the elongated body 16. In the example illustrated at FIG. 6, the embolization device 12 is attached to the elongated body 16 through a connecting member represented by a detach subassembly 50.

The interface member proximal portion 42 is configured to mate with the elongated body distal portion 20. In some examples, the interface member proximal portion 42 defines a protrusion and the elongated body distal portion 20 defines a recess, as illustrated in FIG. 6. In other examples, the interface member proximal portion 42 defines a recess and the elongated body distal portion 20 defines a protrusion. The recess may be configured to receive the protrusion, and the interface member proximal portion 42 and the elongated body distal portion 20 may mate and form a mating connection when the recess receives the protrusion. In some aspects, the recess is a concave surface surrounding an insertion volume, and when the elongated body distal portion 20 mates and forms a mating connection with the interface member proximal portion 42, at least some portion of the protrusion is inserted within the insertion volume.

As illustrated in FIG. 6, the medical assembly 10 may be inserted into the vasculature 30 of a patient. In the example of FIG. 6, the medical assembly 10, including the IMD 12 and the delivery system 14, is received within an inner catheter lumen 52 of the delivery catheter 26. In turn, the delivery catheter 26 is received within the inner guide tube lumen 54 of the guide tube 28. In turn, the guide tube 28 is received within the vasculature (e.g., a vein or vessel) 30 of the patient.

According to the techniques described herein, any or all of the IMD 12, the IMD delivery system 14 (including the elongated body 16 and the interface 22), the delivery catheter 26, and/or the guide tube 28 may include at least one exposed surface that is plasma-treated to improve delivery and/or retrieval of the IMD 12 within the vasculature 30 of the patient. As one example, during insertion of the medical assembly 10 into the vasculature 30 of the patient, one or more exposed surfaces of components of the medical assembly 10, such as the IMD 12, the IMD delivery system 14, the delivery catheter, and/or the guide tube 28, may interface with the vasculature 30 or other components of the medical assembly 10. For example, an outer surface of the guide tube 28 may interface with an inner surface of the vasculature 30, an outer surface of the delivery catheter 26 may interface with an inner surface of the guide tube 28, and an outer surface of the embolization device 12 may interface with an inner surface of the delivery catheter 26. As another example, after insertion of the medical assembly into the vasculature 30 of the patient, one or more exposed surfaces of the medical assembly 10 may interface with biological fluids or tissues at or near the target site of the vasculature 30. For example, an outer surface of the embolization device 12 may interface with blood in or vessel walls of the vasculature 30. To aid or improve delivery, retrieval, retention, and/or operation of the medical assembly 10, one or more exposed surfaces of the medical assembly 10 may be selectively configured, through plasma-treatment processes, with various properties specific to the functions and/or environmental conditions of the particular surface being treated.

The exposed surface includes a metallic surface. For instance, the exposed surface may include one or more of nitinol, titanium, stainless steel, or a cobalt-chromium alloy. In some instances, metallic surfaces may be relatively inert and difficult to functionalize, such as through application of polymer coatings, and such functionalization may weaken the underlying structure or be subject to delamination from shear forces caused by friction.

The plasma-treatment processes described herein may involve application of a plasma to a surface of the medical assembly 10 to chemically and/or physically modify the surface and impart a degree of polarity and/or a degree of roughness to the surface. Without being limited to a particular theory, a plasma may include an ionized or partially ionized gas. The ionized gas interacts with a surface of a substrate, such as the medical assembly 10, to reconfigure a surface structure of the surface, functionalize the surface with reactive groups, or deposit a coating on the surface from the ionized gas. Unlike polymerization techniques, which may result in coatings that do not strongly adhere to the surface, or chemical functionalization techniques, which may be relatively difficult to spatially control, the ionized gas produces modified surfaces that may not include coatings, may include coatings that strongly adhere to the surface, and/or may be spatially controllable.

In some examples, the plasma-treatment processes described herein may impart a degree of polarity to one or more surfaces of the medical assembly 10. A degree of polarity may refer to an amount of polarity from nonpolar (or hydrophobic) to polar (or hydrophilic). As will be explained further below, the degree of polarity may affect lubricity in the presence of polar fluids. For example, a polar (or hydrophilic) surface may promote the adherence of a fluid layer (e.g., blood or other patient fluid, saline, etc.) onto the surface, which may act as a lubricating intermediate layer. The degree of polarity may also affect thrombogenicity of in the presence of blood. For example, a nonpolar (or hydrophobic) surface may promote growth and/or adhesion of proteins (e.g., fibrin) in the blood, thereby forming a clot on the surface.

In some examples, a degree of polarity of a surface may correspond to a wettability/surface tension of the surface as measured by a contact angle with water. For example, a hydrophobic surface may have a relatively high contact angle with water greater than about 90°, such as greater than about 105°, while a hydrophilic surface may have a relatively low contact angle with water less than about 90°, such as less than about 75°. Such contact angle may be measured, for example, using American Society for Testing and Materials (ASTM) D7334 entitled "Standard Practice for Surface Wettability of Coatings, Substrates and Pigments by Advancing Contact Angle Measurement."

In some examples, the plasma-treatment processes described herein imparts a degree of roughness to one or more surfaces of the medical assembly 10. A degree of roughness may refer to an amount and/or amplitude of local surface variations on a surface. As will be explained further below, the degree of roughness may affect lubricity. For example, a smooth (i.e., low surface roughness) surface may reduce a magnitude of friction with other surfaces. The degree of roughness may also affect thrombogenicity or fouling in the presence of blood or tissues. For example, a rough (i.e., high surface roughness) surface may have a relatively high surface area, thereby encouraging deposition of proteins or other tissues involved in clot formation or fouling.

In some examples, a degree of roughness of a surface may correspond to a magnitude, shape, and/or frequency of deviations for a given surface area. As one non-limiting example, a "rough" surface may have a relatively high magnitude of average roughness, such as greater than about 10 micrometers, while a "smooth" surface may have a relatively low magnitude of average roughness, such as less than about 10 micrometers, and a "very smooth" (e.g., very hydrophilic) surface may have deviations averaging less than about 200 nanometers. Such roughness may be measured, for example, using American Society for Mechanical Engineers (ASME) B46.1 entitled "Surface Texture (Surface Roughness, Waviness, and Lay)." However, these example values are merely illustrative and are not intended to be limiting; as used herein, the "roughness" or "smoothness" (e.g., the "hydrophobia" or "hydrophilia") of a plasma-treated surface represents a relative quality of the surface, as compared to the untreated state of the surface.

The following five examples illustrate some non-limiting applications of the plasma-based techniques of this disclosure. These illustrative examples are not mutually exclusive; any or all of these applications may be used in any suitable combination. In some examples, such as the first through fourth examples described below, one or more exposed surfaces of the medical assembly 10 may be plasma-treated to modify a lubricity of the one or more exposed surfaces. For example, plasma-treatment of a surface may increase a polarity (i.e., hydrophilicity) of the surface and/or decrease a roughness of the surface, such that the surface may have increased lubricity. In some examples, such as the fifth example described below, one or more exposed surfaces of the medical assembly 10 may be plasma-treated to modify a thrombogenicity of the one or more exposed surfaces. For example, plasma-treatment of a surface may decrease a polarity (i.e., hydrophobicity) of the surface and/or increase a roughness of the surface, such that the surface may have increased thrombogenicity.

In a first example of the plasma-based techniques of this disclosure, an exterior sheath surface 28A of the guide tube 28 may be treated with a plasma-based treatment in order to reduce friction between the exterior sheath surface 28A and an interior vessel surface 30A while the guide tube 28 is introduced distally into and/or proximally withdrawn from the vasculature 30 of patient 40. For instance, as detailed further below, the exterior sheath surface 28A may be plasma-treated so as to increase a relative degree of hydrophilia of the exterior sheath surface 28A, thereby promoting the adherence of a fluid layer (e.g., blood or other patient fluid, saline, etc.) onto the exterior sheath surface 28A. The fluid layer then acts as a lubricating intermediate layer between the exterior sheath surface 28A and the interior vessel surface 30A while the two surfaces are driven past one another. In this way, plasma-treatment processes described herein may reduce friction between the exterior sheath surface 28A and the interior vessel surface 30A, and correspondingly reduce the delivery force to advance the guide tube 28 through the vasculature 30.

Similarly, in a second example of the plasma-based techniques of this disclosure, either or both of an interior sheath surface 28B and an exterior catheter surface 26A are treated with a plasma-based treatment process in order to reduce an amount of friction associated with contact between the two surfaces while the delivery catheter 26 is introduced distally into, and/or proximally withdrawn from, the inner sheath lumen 54 of the guide tube 28. For instance, in one such example, both of the interior sheath surface 28B and the exterior catheter surface 26A may be treated with similar plasma-based treatment processes (as detailed further below) in order to impart or enhance hydrophilic properties of the respective surfaces. In an alternate example, a first surface of the two surfaces may be plasma-treated with a first plasma-treatment process to impart hydrophilia onto, or enhance the hydrophilia of, the first surface; a second surface of the two surfaces may be plasma treated with a second plasma-treatment process to impart hydrophobia onto, or enhance the hydrophobia of, the second surface. In such examples, a lubricating fluid layer may tend to adhere to the first (hydrophilic) surface, causing the second (hydrophobic) surface to repel the lubricated first surface, thereby reducing an amount of friction as the first surface moves past the second surface, or vice versa. In these ways, plasma-treatment processes described herein may reduce friction between the exterior catheter surface 26A and the interior sheath surface 28B, and correspondingly reduce the delivery force to advance the delivery catheter 26 through the guide tube 28.

In a third example of the plasma-based techniques of this disclosure, the guide tube 28 is not present, or alternatively, the guide tube distal end 32 does not extend fully to the target site 34, such that the delivery catheter 26 extends distally outward from the guide tube distal end 32. In such examples, the exterior catheter surface 26A of the delivery catheter 26 may be treated with a plasma-based treatment process (as described above, and as detailed further below) in order to reduce friction between the exterior catheter surface 26A and the interior vessel surface 30A. In this way, plasma-treatment processes described herein may reduce friction between the exterior catheter surface 26A and the interior vessel surface 30A, and correspondingly reduce the delivery force to advance the delivery catheter 26 through the vasculature 30 to the target site 34.

In a fourth example of the plasma-based techniques of this disclosure, either or both of an interior catheter surface 26B and an exterior surface of the medical assembly 10 are treated with a plasma-based treatment process in order to reduce an amount of friction associated with contact between the two surfaces while the IMD 12 is introduced distally into, and/or proximally withdrawn from, the inner catheter lumen 52 of the catheter 26. For instance, in one such example, the interior catheter surface 26B, and both/either the exterior elongated body surface 16A and/or the exterior IMD surface 12A, may be treated with similar plasma-based treatment processes (as detailed further below) in order to impart or enhance hydrophilic properties of the respective surfaces. In an alternate example, a first surface of the two surfaces may be plasma-treated with a first plasma-treatment process to impart hydrophilia onto, or enhance the hydrophilia of, the first surface; a second surface of the two surfaces may be plasma treated with a second plasma-treatment process to impart hydrophobia onto, or enhance the hydrophobia of, the second surface. In such examples, a lubricating fluid layer may tend to adhere to the first (hydrophilic) surface, causing the second (hydrophobic) surface to repel the lubricated first surface, thereby reducing an amount of friction as the first surface moves past the second surface, or vice versa. In this way, plasma-treatment processes described herein may reduce friction between the IMD 12 (e.g., the exterior elongated body surface 16A and/or the exterior IMD surface 12A) and the interior catheter surface 26B, and correspondingly reduce the delivery force to advance the IMD 12 through the delivery catheter 26.

In a fifth example of the plasma-based techniques of this disclosure, the exterior IMD surface 12A of the embolization device 12 may be treated with a plasma-based treatment process (as described above, and as detailed further below) in order to impart hydrophobic properties onto, or enhance hydrophobic properties of, the exterior IMD surface 12A. For instance, promoting a hydrophobia of exterior IMD surface 12A may tend to produce thrombogenic effects of the exterior IMD surface 12A. Accordingly, once IMD 12 is positioned within target treatment site 34 (as depicted in FIG. 5), the imparted thrombogenic properties may increase a tendency of a desired blood clot to develop and solidify (e.g., coagulate) around IMD 12, thereby retaining the IMD 12 in place at the target treatment site 34 without (or with reduced) additional medical intervention. In other examples, such as when IMD 12 includes a device other than an embolization device, exterior IMD surface 12A may be plasma-treated to make the surface more hydrophilic. In this way, plasma-treatment processes described herein may increase clotting at the IMD 12 (e.g., the exterior IMD surface 12A), and correspondingly increase the retention of the IMD 12 at the target site 34.

In any of the above examples, the respective plasma-based treatment process may include any suitable plasma treatment configured to produce the desired effects (e.g., surface properties). In other words, the plasma-treatments described herein may include additive plasma treatments, subtractive plasma treatments, a plasma treatment that is both additive and subtractive, or any suitable combination thereof.

As used herein, "additive" plasma treatments include any use of a plasma to adhere material to an exposed surface of an intravascular medical assembly, including to polish the exposed surface to reduce friction associated with the surface, to adhere a desired coating to the exposed surface, or both. For instance, an additive plasma treatment may function to "polish" an exposed surface by at least partially "filling in" existing divots, valleys, gaps, or other similar "negative space" (e.g., radially inward or concave) deviations from a theoretical smooth surface. In some such examples, the plasma-treatment process may smooth out the exposed surface by redistributing portions of an existing outer-most layer of material so as to "average out" the deviations into a smoother surface having a common chemical makeup. In other such examples, the plasma-treatment process may smooth out the exposed surface by depositing and adhering newly added material into the negative-space deviations. The newly added material may have the same chemical composition as the existing exposed surface, or may have a different chemical composition.

In additional or alternative examples, an "additive" plasma-treatment process may function to form and adhere a "coating," or outer-most layer, onto the exposed surface as a whole (as compared to just the negative-space deviations), wherein the coating includes one or more desired physical properties, such as hydrophilia, hydrophobia, or thrombogenicity. For example, as will be explained further below, a reactive plasma, such as a nitrogen and/or oxygen plasma, may be used to deposit a coating that includes nitrogen and/or oxygen onto the exposed surface. Because the added coating may function simultaneously to fill in the negative-space surface deviations as described above, the term "additive" is used herein to encompass both applications. For examples involving metallic surfaces, one example of an additive process is electroplating.

As used herein, "subtractive" plasma treatments include any use of a plasma to remove material from an exposed surface of an intravascular medical assembly, including to polish the exposed surface to reduce friction associated with the surface, to remove an outermost layer from the exposed surface, or both. For instance, a subtractive plasma treatment may function to "polish" an exposed surface by at least partially wearing down or removing existing bumps or other similar "positive space" (e.g., radially outward or convex) deviations from a theoretical smooth surface. In some examples herein, a plasma-based polishing technique may simultaneously be both additive and subtractive, such as when the plasma treatment functions to smooth out an exposed surface by removing material from bumps in the surface and redistributing the removed material to fill in divots in the surface.

Accordingly, the plasma-based treatments of this disclosure, including those described with respect to the five illustrative examples above, may include electrolytic plasma-polishing (or "electropolishing") of an exposed metal surface of an intravascular medical assembly. As used herein, "electrolytic plasma-polishing" refers to the process in which a metallic portion of the medical assembly is anodically polarized, placed in an electrolytic solution (e.g., viscous, acidic solutions, such as sulfuric and/or phosphoric acid), and subjected to an electric current. In response, excess regions of metal on the exposed surface are oxidized and dissolved in the electrolyte, thereby reducing variability in the exposed surface and reducing friction otherwise resulting from subsequent physical contact with the surface.

In some such examples, or in alternative examples, the exposed metal surface of the intravascular medical assembly may be electropolished in the presence of a reactive gas to (further) reduce the surface variability, as detailed further below. Additionally or alternatively, the plasma-based treatment may include plasma-coating the exposed surface with a substance formed from the reactive gas in order to impart the desired properties of hydrophilia, hydrophobia, and/or thrombogenicity, as appropriate, onto the surface.

As one non-limiting example, plasma-based treatments in accordance with techniques of this disclosure includes electrolytic plasma-polishing of an exposed surface of an intravascular medical assembly in the presence of hydrogen and oxygen gas. In some such examples, the plasma-polishing process may simultaneously, and at least temporarily, plasma-coat the exposed surface with an outermost layer formed from a chemical that comprises a hydroxyl group. Because hydroxyl-based coatings may tend to exhibit substantially hydrophilic properties, such plasma-based treatments are desirable for further reducing friction associated with introducing or withdrawing medical assembly 10 (including the newly coated surface) within the vasculature 30 of the patient.

As another non-limiting example, plasma-based treatments in accordance with techniques of this disclosure include electrolytic plasma-polishing of an exposed surface of an intravascular medical assembly in the presence of nitrogen and oxygen gas. In some such examples, the plasma-polishing process may simultaneously, and at least temporarily, plasma-coat the exposed surface with an outermost layer formed from a chemical that at least includes nitrogen oxide. Because some nitrogen-oxide-based coatings tend to exhibit hydrophilic properties, such plasma-based treatments are desirable for further reducing friction associated with introducing or withdrawing medical assembly 10 (including the newly coated surface) within the vasculature 30 of the patient.

The techniques of this disclosure further include first, determining a desired relative degree of a certain physical or chemical property (e.g., hydrophilia, hydrophobia, and/or thrombogenicity), determining a corresponding customized plasma-treatment process that achieves the desired physical or chemical property, and then modifying one or more variable parameters of the plasma-treatment process to produce the desired physical or chemical property. The variable parameters of the plasma-treatment process may include, as non-limiting examples, a chemical composition of a substance involved in the plasma-treatment process, a pulse frequency of an electric current driving the plasma-treatment process, and a fluid pressure of the electrolyte and/or the surrounding environment.

For instance, in the example above, in which the exposed surface is plasma-coated with a nitrogen-oxide compound, depending on the desired level of hydrophilia of the exposed surface, the plasma-treatment may be conducted in the presence of a corresponding customizable ratio of nitrogen gas to oxygen gas. As one illustrative example, the exposed surface of the medical assembly may be plasma-treated in the presence of a nitrogen-oxygen mixture that is selected to have a ratio of about 4 to 1. Conversely, to produce a plasma coating having the "opposite" physical properties (or increased or reduced physical properties), the exposed surface of the medical assembly may be plasma-treated in the presence of a nitrogen-oxygen gas ratio of about 1 to 4. However, these example values are merely illustrative of the concept of modifying surface properties by selecting an appropriate ratio of chemicals, and are not intended to be limiting.

As another illustrative example, the exposed surface of the medical assembly may be plasma-treated at a fluid pressure that is selected to be at or about atmospheric pressure. In other examples, the surface may be plasma-treated under an increased fluid pressure or a decreased fluid pressure, as desired.

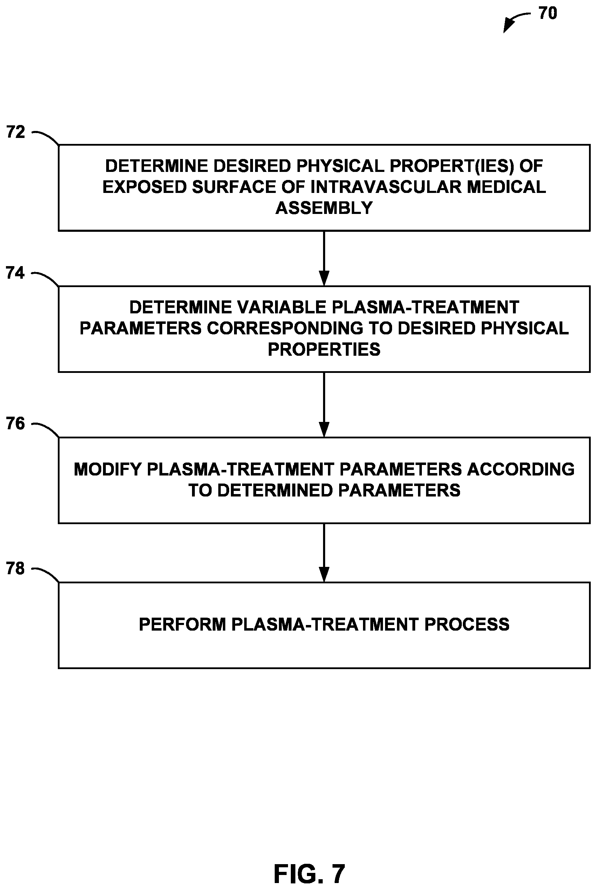

FIG. 7 is a flow diagram of an example method 70 of plasma-treating at least one exposed surface of an intravascular medical assembly, such as the medical assembly 10 of FIG. 1, in accordance with techniques of this disclosure. The method includes determining a desired physical or chemical property for the exposed surface of the medical assembly (72). For example, the method may include determining a desired bulk surface property of the exposed surface of a portion of the medical assembly, such as lubricity, thrombogenicity, anti-thrombogenicity, and/or anti-fouling, and determining a desired physical or chemical property that corresponds to the desired bulk surface property. For instance, in examples in which the exposed surface includes an exterior surface 28A (FIG. 6) of an intravascular guide tube or sheath 28, a desired physical property may include a degree of hydrophilia to reduce friction between the exterior sheath surface 28A and an interior surface 30A of a vessel of a patient's vasculature. Additionally or alternatively, in examples in which the exposed surface includes an exterior surface 12A of an embolization device 12 configured to occlude a cavity at a target site 34 within the patient's vasculature, a desired physical property may include degrees of hydrophobia and/or thrombogenicity to help retain the embolization device 12 in place at the target treatment site 34.

In response to determining one or more desired physical or chemical properties for at least one exposed surface, the method further includes determining values for one or more variable or customizable parameters of a plasma-treatment process, such that the plasma-treatment process produces the desired physical properties (74). For instance, the variable parameters may include a chemical composition of a substance to be ionized, a pulse frequency of an electrical current, or a fluid pressure, as non-limiting examples. The method further includes, responsive to determining the corresponding values, selecting or adjusting the parameters to produce the corresponding values (76).

The method further includes plasma-treating the exposed surface of the intravascular medical assembly to produce the desired physical properties (78). In examples described herein, the plasma-treatment process may include both electrolytic-polishing of the exposed surface in the presence of a reactive gas to reduce variability in the metal surface and reduce friction, and plasma-coating the metal surface with a substance formed from the reactive gas, wherein the substance imparts the desired physical properties onto the exposed surface.

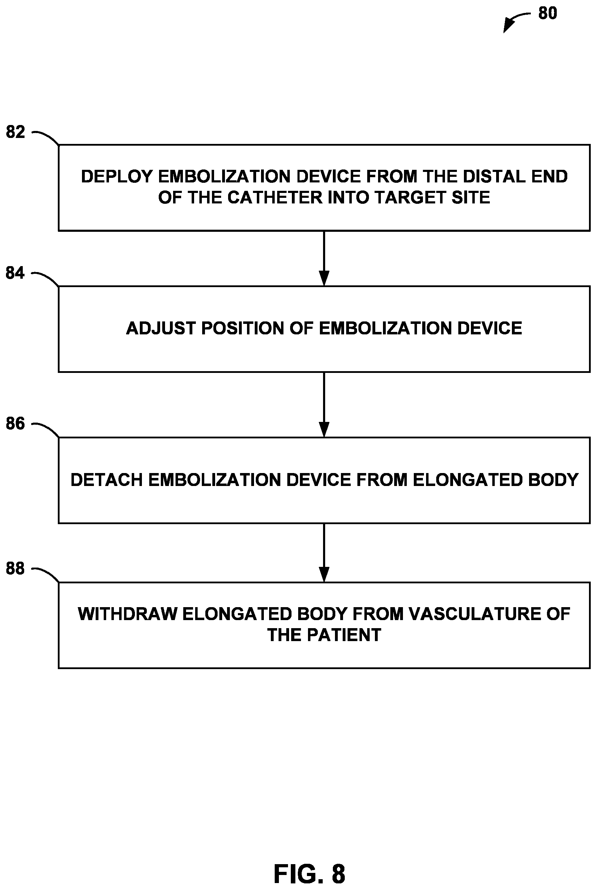

FIG. 8 is a flow diagram of an example method 80 of using an intravascular medical assembly, such as the medical assembly 10 of FIG. 1, that includes at least one exposed surface that has been plasma-treated to improve use of the medical assembly 10. In some examples, the embolization device 12, the interface member 22, and the elongated body 16 are preloaded into the inner catheter lumen 52 of the catheter 26 by a distributor of the embolization device 12. In other examples, a clinician may introduce the embolization device 12, the interface member 22, and the elongated body 16 into the inner catheter lumen 52 of the catheter 26. A clinician may position a distal portion of the catheter 26 proximate the target site 34 and advance the embolization device 12 through the inner catheter lumen 52 to at least partially deploy the embolization device 12 at the target site 34 within a patient 40 (82). For example, the clinician may apply a pushing force to the elongated body 16 (e.g., to the elongated body proximal portion 18), which is transmitted through the mating connection to the interface member 22 and the embolization device 12. The clinician may continue to advance the delivery system 14 toward the distal end of the catheter 26, until the embolization device 12 is at least partially deployed from the inner catheter lumen 52 and to the target site 34.

In accordance with techniques of this disclosure, one or more exposed surfaces of the delivery system 14 may be plasma-treated to reduce friction, and thereby the pushing force, associated with advancing the delivery system through the inner catheter lumen 52 of the delivery catheter 26. For instance, the exterior surface 16A of the elongated body 16 of the delivery system 14 may have been both electrolytic-plasma-polished to smooth out the exterior surface 16A, as well as plasma-coated with a hydrophilic coating to attract a lubricating fluid layer toward the exterior surface 16A, thereby reducing the friction created while advancing the delivery system 14 through the inner catheter lumen 52.

In some examples, the clinician may adjust the position of the embolization device 12 relative to the target site 34 (84). For example, the clinician may manipulate the position of the embolization device 12 to position the embolization device 12 as desired at the target site 34, by maintaining or establishing mating between the elongated body distal portion 20 and the interface member proximal portion 42, then applying an axial or non-axial force to the elongated body proximal portion 18 and transmitting the force from the elongated body proximal portion 18 through the mating connection and to interface member 22 and embolization device 12, until the force generates motion of embolization device 12 in the target site 34.

After embolization device 12 is positioned as desired at target site 34, the clinician may detach embolization device 12 from elongated body 16 (86). For example, the clinician may facilitate detachment by retracting cord 62 and allowing ball 64 to pass through delivery-system inner lumen 66 (FIG. 6), or by using another appropriate methodology. After embolization device 12 is detached (and separated) from elongated body 16, the clinician may proximally withdraw elongated body 16 from the vasculature of the patient, e.g., through lumen 52 of catheter 26, while embolization device 12 and interface member 22 remain at target site 34 (88).

In accordance with techniques of this disclosure, one or more exposed surfaces of the embolization device 12 may be plasma-treated to increase thrombogenicity, and thereby the retention, associated with positioning the embolization device 12 at the target site 34. For instance, the exterior surface of the embolization device 12 may have been plasma-coated with a hydrophobic coating to increase a rate of accumulation of proteins on the exterior surface, thereby increasing clot formation.

Various examples have been described. These and other examples are within the scope of the following claims.

## Claims

1. A medical assembly comprising:
an elongated structure configured to deliver or retrieve a medical device within vasculature of a patient, wherein the elongated structure comprises an exposed surface comprising a metal surface, the exposed surface being treated with a plasma-treatment process using a reactive gas to reduce friction associated with the surface, and wherein the exposed surface includes a hydrophilic substance formed from the reactive gas,
wherein the plasma-treatment process comprises electrolytic-polishing of the surface in the presence of the reactive gas to reduce variability in the surface,
wherein the reactive gas comprises a mixture of nitrogen and oxygen,
wherein the hydrophilic substance comprises one or more groups comprising nitrogen and oxygen, and
wherein the mixture of nitrogen and oxygen comprises a nitrogen-oxygen ratio of greater than or equal to about 4:1.

2. The medical assembly of claim 1, wherein the exposed surface has a contact angle less than or equal to about 90°.

3. The medical assembly of claim 1 or 2, wherein the exposed surface has a surface roughness less than about 10 microns.

4. The medical assembly of any of claims 1 to 3, wherein the plasma-treatment process comprises a first plasma-treatment process,
wherein the medical assembly further comprises the medical device, and wherein an exposed surface of the medical device is treated with a second plasma-treatment process,
wherein the exposed surface of the medical device includes a hydrophobic substance formed from a reactive gas of the second plasma-treatment process, such that the hydrophobic substance is configured to attract and coagulate blood of the patient around the medical device to retain the medical device in place in the vasculature of the patient.

5. The medical device of claim 4, wherein the second plasma-treatment process is substantially the same as the first plasma-treatment process.

6. The medical assembly of any of claims 1 to 5, wherein the medical device comprises an interventional element, wherein the elongated structure comprises an intravascular-insertable shaft removably coupled to the interventional element.

7. The medical assembly of claim 6, wherein the medical device comprises a thrombectomy device or a clot-grabbing device, and wherein the interventional element is positioned at a distal portion of the elongated structure, and/or
wherein the interventional element comprises a balloon-expandable device, an energy-emitting device, an energy-delivery device, an electrode, a heating coil, a fiber-optic device, an electrical source, and/or an ultrasonic source.

8. The medical assembly of any of claims 1 to 7, wherein the medical assembly further comprises a medical device delivery system comprising a coil-delivery system, a stent-delivery system, a flow-diverter-delivery system, a cardiac-pacing-device-delivery system, or a heart-valve-delivery system.

9. The medical assembly of any of claims 1 to 8, wherein the elongated structure defines a shaft length configured to enable neurovascular access for the elongated structure, and
wherein the shaft length is greater than one meter, preferably about 160 centimeters (cm).

10. The medical assembly of any of claims 1 to 9, wherein the elongated structure defines a shaft length configured to enable coronary access, cardiac access, or peripheral access.

11. The medical assembly of any of claims 1 to 3, wherein the plasma-treatment process comprises a first plasma-treatment process to form a first hydrophilic substance,
wherein the medical assembly further comprises a sheath comprising an inner lumen configured to receive the elongated structure while the sheath is positioned within the vasculature of the patient, and
wherein an interior surface of the sheath is treated with a second plasma-treatment process to form a second hydrophilic substance,
optionally wherein an exterior surface of the sheath is treated with a third plasma-treatment process, and
wherein the third plasma-treatment process is substantially the same as the first plasma treatment process.

12. The medical assembly of any of claim 11, wherein the first plasma-treatment process and the second plasma-treatment process reduce a friction between the surface of the elongated structure and the interior surface of the sheath.

13. The medical assembly of any of claims 1 to 12, wherein the elongated structure comprises one or more of a core wire, a hypotube, or a filament,
wherein the elongated structure comprises the hypotube, and
wherein the hypotube comprises a laser-cut hypotube, a spiral-cut hypotube, or a slotted-cut hypotube.

14. The medical assembly of any of claims 1 to 13, wherein the surface comprises one or more of nitinol, titanium, stainless steel, or a cobalt-chromium alloy.

15. A method comprising:
applying a plasma-treatment process using a reactive gas to an exposed surface of an elongated structure of a medical assembly to change at least one of a polarity or a surface roughness of the exposed surface, wherein the exposed surface includes a hydrophilic substance formed from the reactive gas, wherein applying the plasma-treatment process further comprises:
electrolytic-polishing of the metal surface in the presence of the reactive gas to reduce the surface roughness of the exposed surface; and
plasma-coating the surface with a hydrophilic substance formed from the reactive gas,
wherein the plasma-treatment process is applied to the elongated structure at atmospheric pressure,
wherein the plasma-treatment process further comprises:
selecting an electric-pulse frequency associated with a desired degree of hydrophilia of the surface; and
plasma-coating the metal surface according to the selected electric-pulse frequency,
wherein the reactive gas comprises a mixture of nitrogen and oxygen,
wherein the hydrophilic substance comprises one or more groups comprising nitrogen and oxygen, and
wherein the mixture of nitrogen and oxygen comprises a nitrogen-oxygen ratio of greater than or equal to about 4:1.

## Patentansprüche

1. Medizinische Anordnung, umfassend:
eine längliche Struktur, die konfiguriert ist, um eine medizinische Vorrichtung innerhalb des Gefäßsystems eines Patienten zuzuführen oder zurückzuholen, wobei die längliche Struktur eine freiliegende Oberfläche umfasst, die eine Metalloberfläche umfasst, wobei die freiliegende Oberfläche mit einem Plasmabehandlungsprozess unter Verwendung eines reaktiven Gases behandelt ist, um die mit der Oberfläche verbundene Reibung zu verringern, und wobei die freiliegende Oberfläche eine hydrophile Substanz einschließt, die aus dem reaktiven Gas gebildet ist,
wobei der Plasmabehandlungsprozess elektrolytisches Polieren der Oberfläche in Gegenwart des reaktiven Gases umfasst, um die Variabilität in der Oberfläche zu verringern,
wobei das reaktive Gas ein Gemisch aus Stickstoff und Sauerstoff umfasst,
wobei die hydrophile Substanz eine oder mehrere Gruppen umfasst, die Stickstoff und Sauerstoff umfassen, und
wobei das Gemisch aus Stickstoff und Sauerstoff ein Stickstoff-Sauerstoff-Verhältnis von größer als oder gleich etwa 4:1 umfasst.

2. Medizinische Anordnung nach Anspruch 1, wobei die freiliegende Oberfläche einen Kontaktwinkel von kleiner als oder gleich etwa 90° aufweist.

3. Medizinische Anordnung nach Anspruch 1 oder 2, wobei die freiliegende Oberfläche eine Oberflächenrauheit von weniger als etwa 10 Mikrometern aufweist.

4. Medizinische Anordnung nach einem der Ansprüche 1 bis 3, wobei der Plasmabehandlungsprozess einen ersten Plasmabehandlungsprozess umfasst,
wobei die medizinische Anordnung ferner die medizinische Vorrichtung umfasst, und wobei eine freiliegende Oberfläche der medizinischen Vorrichtung mit einem zweiten Plasmabehandlungsprozess behandelt wird,
wobei die freiliegende Oberfläche der medizinischen Vorrichtung eine hydrophobe Substanz umfasst, die aus einem reaktiven Gas des zweiten Plasmabehandlungsprozesses gebildet ist, sodass die hydrophobe Substanz konfiguriert ist, um Blut des Patienten um die medizinische Vorrichtung herum anzuziehen und zu koagulieren, um die medizinische Vorrichtung im Gefäßsystem des Patienten an Ort und Stelle zu halten.

5. Medizinische Vorrichtung nach Anspruch 4, wobei der zweite Plasmabehandlungsprozess im Wesentlichen der gleiche ist wie der erste Plasmabehandlungsprozess.

6. Medizinische Anordnung nach einem der Ansprüche 1 bis 5, wobei die medizinische Vorrichtung ein interventionelles Element umfasst, wobei die längliche Struktur einen intravaskulär einführbaren Schaft umfasst, der lösbar mit dem interventionellen Element gekoppelt ist.

7. Medizinische Anordnung nach Anspruch 6, wobei die medizinische Vorrichtung eine Thrombektomievorrichtung oder eine Gerinnselgreifvorrichtung umfasst, und wobei das interventionelle Element an einem distalen Abschnitt der länglichen Struktur positioniert ist, und/oder
wobei das interventionelle Element eine ballonexpandierbare Vorrichtung, eine energieemittierende Vorrichtung, eine energiezuführende Vorrichtung, eine Elektrode, eine Heizspule, eine faseroptische Vorrichtung, eine Stromquelle und/oder eine Ultraschallquelle umfasst.

8. Medizinische Anordnung nach einem der Ansprüche 1 bis 7, wobei die medizinische Anordnung ferner ein Zuführsystem für eine medizinische Vorrichtung umfasst, umfassend ein Coil-Zuführsystem, ein Stent-Zuführsystem, ein Flow-Diverter-Zuführsystem, ein Herzschrittmachervorrichtungs-Zuführsystem oder ein Herzklappen-Zuführsystem.

9. Medizinische Anordnung nach einem der Ansprüche 1 bis 8, wobei die längliche Struktur eine Schaftlänge definiert, die konfiguriert ist, um einen neurovaskulären Zugang für die längliche Struktur zu ermöglichen, und
wobei die Schaftlänge größer als ein Meter ist, vorzugsweise etwa 160 Zentimeter (cm).

10. Medizinische Anordnung nach einem der Ansprüche 1 bis 9, wobei die längliche Struktur eine Schaftlänge definiert, die konfiguriert ist, um einen koronaren Zugang, einen kardialen Zugang oder einen peripheren Zugang zu ermöglichen.

11. Medizinische Anordnung nach einem der Ansprüche 1 bis 3, wobei der Plasmabehandlungsprozess einen ersten Plasmabehandlungsprozess umfasst, um eine erste hydrophile Substanz zu bilden,
wobei die medizinische Anordnung ferner eine Hülse umfasst, die ein inneres Lumen umfasst, das konfiguriert ist, um die längliche Struktur aufzunehmen, während die Hülse innerhalb des Gefäßsystems des Patienten positioniert ist, und
wobei eine innere Oberfläche der Hülse mit einem zweiten Plasmabehandlungsprozess behandelt wird, um eine zweite hydrophile Substanz zu bilden,
wobei optional eine äußere Oberfläche der Hülse mit einem dritten Plasmabehandlungsprozess behandelt wird, und
wobei der dritte Plasmabehandlungsprozess im Wesentlichen der gleiche ist wie der erste Plasmabehandlungsprozess.

12. Medizinische Anordnung nach Anspruch 11, wobei der erste Plasmabehandlungsprozess und der zweite Plasmabehandlungsprozess eine Reibung zwischen der Oberfläche der länglichen Struktur und der inneren Oberfläche der Hülse verringern.

13. Medizinische Anordnung nach einem der Ansprüche 1 bis 12, wobei die längliche Struktur eines oder mehrere von einem Kerndraht, einem Hypotube oder einem Filament umfasst,
wobei die längliche Struktur das Hypotube umfasst, und
wobei das Hypotube ein lasergeschnittenes Hypotube, ein spiralgeschnittenes Hypotube oder ein schlitzgeschnittenes Hypotube umfasst.

14. Medizinische Anordnung nach einem der Ansprüche 1 bis 13, wobei die Oberfläche eines oder mehrere von Nitinol, Titan, Edelstahl oder einer Kobalt-Chrom-Legierung umfasst.

15. Verfahren, umfassend:
Anwenden eines Plasmabehandlungsprozesses unter Verwendung eines reaktiven Gases auf eine freiliegende Oberfläche einer länglichen Struktur einer medizinischen Anordnung, um mindestens eines von einer Polarität oder einer Oberflächenrauheit der freiliegenden Oberfläche zu ändern, wobei die freiliegende Oberfläche eine hydrophile Substanz einschließt, die aus dem reaktiven Gas gebildet ist, wobei das Anwenden des Plasmabehandlungsprozesses ferner umfasst:
elektrolytisches Polieren der Metalloberfläche in Gegenwart des reaktiven Gases, um die Oberflächenrauheit der freiliegenden Oberfläche zu verringern; und
Plasmabeschichten der Oberfläche mit einer aus dem reaktiven Gas gebildeten hydrophilen Substanz, wobei der Plasmabehandlungsprozess bei atmosphärischem Druck auf die längliche Struktur angewendet wird,
wobei der Plasmabehandlungsprozess ferner umfasst:
Auswählen einer elektrischen Impulsfrequenz, die mit einem gewünschten Grad an Hydrophilie der Oberfläche verbunden ist; und
Plasmabeschichten der Metalloberfläche gemäß der ausgewählten elektrischen Impulsfrequenz,
wobei das reaktive Gas ein Gemisch aus Stickstoff und Sauerstoff umfasst,
wobei die hydrophile Substanz eine oder mehrere Gruppen umfasst, die Stickstoff und Sauerstoff umfassen, und
wobei das Gemisch aus Stickstoff und Sauerstoff ein Stickstoff-Sauerstoff-Verhältnis von größer als oder gleich etwa 4:1 umfasst.

## Revendications

1. Ensemble médical comprenant :
une structure allongée conçue pour administrer ou récupérer un dispositif médical à l'intérieur d'un système vasculaire d'un patient, dans lequel la structure allongée comprend une surface exposée comprenant une surface métallique, la surface exposée étant traitée par un procédé de traitement au plasma à l'aide d'un gaz réactif pour réduire la friction associée à la surface, et dans lequel la surface exposée comporte une substance hydrophile formée à partir du gaz réactif,
dans lequel le procédé de traitement au plasma comprend le polissage électrolytique de la surface en présence du gaz réactif pour réduire la variabilité de la surface,
dans lequel le gaz réactif comprend un mélange d'azote et d'oxygène,
dans lequel la substance hydrophile comprend un ou plusieurs groupes comprenant de l'azote et de l'oxygène, et
dans lequel le mélange d'azote et d'oxygène comprend un rapport azoteoxygène supérieur ou égal à environ 4:1.

2. Ensemble médical selon la revendication 1, dans lequel la surface exposée a un angle de contact inférieur ou égal à environ 90 °.

3. Ensemble médical selon la revendication 1 ou 2, dans lequel la surface exposée a une rugosité de surface inférieure à environ 10 microns.

4. Ensemble médical selon l'une quelconque des revendications 1 à 3, dans lequel le procédé de traitement au plasma comprend un premier procédé de traitement au plasma,
dans lequel l'ensemble médical comprend en outre le dispositif médical, et dans lequel une surface exposée du dispositif médical est traitée avec un deuxième procédé de traitement au plasma,
dans lequel la surface exposée du dispositif médical comporte une substance hydrophobe formée à partir d'un gaz réactif du deuxième procédé de traitement au plasma, de telle sorte que la substance hydrophobe est conçue pour attirer et coaguler le sang du patient autour du dispositif médical afin de retenir le dispositif médical en place dans le système vasculaire du patient.

5. Dispositif médical selon la revendication 4, dans lequel le deuxième procédé de traitement au plasma est sensiblement le même que le premier procédé de traitement au plasma.

6. Ensemble médical selon l'une quelconque des revendications 1 à 5, dans lequel le dispositif médical comprend un élément d'intervention, dans lequel la structure allongée comprend une tige insérable par voie intravasculaire accouplée de manière amovible à l'élément d'intervention.

7. Ensemble médical selon la revendication 6, dans lequel le dispositif médical comprend un dispositif de thrombectomie ou un dispositif de capture de caillots, et dans lequel l'élément d'intervention est positionné au niveau d'une partie distale de la structure allongée, et/ou
dans lequel l'élément interventionnel comprend un dispositif expansible par ballonnet, un dispositif d'émission d'énergie, un dispositif d'administration d'énergie, une électrode, une bobine de chauffage, un dispositif à fibre optique, une source électrique et/ou une source à ultrasons.

8. Ensemble médical selon l'une quelconque des revendications 1 à 7, dans lequel l'ensemble médical comprend en outre un système d'administration de dispositif médical comprenant un système d'administration de bobine, un système d'administration d'endoprothèse, un système d'administration de déviateur de flux, un système d'administration de dispositif de stimulation cardiaque ou un système d'administration de valvule cardiaque.

9. Ensemble médical selon l'une quelconque des revendications 1 à 8, dans lequel la structure allongée définit une longueur de tige conçue pour permettre un accès neurovasculaire pour la structure allongée, et
dans lequel la longueur de tige est supérieure à un mètre, de préférence environ 160 centimètres (cm).

10. Ensemble médical selon l'une quelconque des revendications 1 à 9, dans lequel la structure allongée définit une longueur de tige conçue pour permettre un accès coronaire, un accès cardiaque ou un accès périphérique.

11. Ensemble médical selon l'une quelconque des revendications 1 à 3, dans lequel le procédé de traitement au plasma comprend un premier procédé de traitement au plasma pour former une première substance hydrophile,
dans lequel l'ensemble médical comprend en outre une gaine comprenant une lumière interne conçue pour recevoir la structure allongée lorsque la gaine est positionnée à l'intérieur du système vasculaire du patient, et
dans lequel une surface intérieure de la gaine est traitée avec un deuxième procédé de traitement au plasma pour former une seconde substance hydrophile,
éventuellement dans lequel une surface extérieure de la gaine est traitée avec un troisième procédé de traitement au plasma, et
dans lequel le troisième procédé de traitement au plasma est sensiblement le même que le premier procédé de traitement au plasma.

12. Ensemble médical selon l'une quelconque des revendications 11, dans lequel le premier procédé de traitement au plasma et le deuxième procédé de traitement au plasma réduisent une friction entre la surface de la structure allongée et la surface intérieure de la gaine.

13. Ensemble médical selon l'une quelconque des revendications 1 à 12, dans lequel la structure allongée comprend un ou plusieurs parmi un fil central, un tube hypodermique ou un filament,
dans lequel la structure allongée comprend le tube hypodermique, et
dans lequel le tube hypodermique comprend un tube hypodermique découpé au laser, un tube hypodermique découpé en spirale ou un tube hypodermique découpé en fentes.

14. Ensemble médical selon l'une quelconque des revendications 1 à 13, dans lequel la surface comprend un ou plusieurs parmi le nitinol, le titane, l'acier inoxydable, ou un alliage cobalt-chrome.

15. Procédé comprenant :
l'application d'un procédé de traitement au plasma à l'aide d'un gaz réactif sur une surface exposée d'une structure allongée d'un ensemble médical pour modifier au moins l'une parmi une polarité ou une rugosité de surface de la surface exposée, dans lequel la surface exposée comporte une substance hydrophile formée à partir du gaz réactif, dans lequel l'application du processus de traitement au plasma comprend en outre :
le polissage électrolytique de la surface métallique en présence du gaz réactif pour réduire la rugosité de surface de la surface exposée ; et
le revêtement de la surface par plasma avec une substance hydrophile formée à partir du gaz réactif, dans lequel le procédé de traitement par plasma est appliqué à la structure allongée à pression atmosphérique,
dans lequel le procédé de traitement au plasma comprend en outre :
la sélection d'une fréquence d'impulsion électrique associée à un degré souhaité d'hydrophilie de la surface ; et
le revêtement plasma de la surface métallique selon la fréquence d'impulsion électrique choisie,
dans lequel le gaz réactif comprend un mélange d'azote et d'oxygène,
dans lequel la substance hydrophile comprend un ou plusieurs groupes comprenant de l'azote et de l'oxygène, et
dans lequel le mélange d'azote et d'oxygène comprend un rapport azoteoxygène supérieur ou égal à environ 4:1.
